# EUROPEAN PATENT SPECIFICATION

(11) **EP 2 418 288 B1**
(45) Date of publication and mention of the grant of the patent: **28.11.2012**
(21) Application number: 10382224.3
(22) Date of filing: 09.08.2010
(51) Int. Cl.: C12Q 1/68

(54) **Method for the diagnosis and prognosis of tolerance in liver transplantation employing liver tissue**
Verfahren zur Diagnose und Prognose der Toleranz bei der Lebertransplantation unter Verwendung von Lebergewebe
Procédé pour le diagnostic et le pronostic de la tolérance dans la greffe de foie au moyen de tissus hépatiques

(43) Date of publication of application: 15.02.2012
(73) Proprietor: Hospital Clínic de Barcelona, 08036 Barcelona (ES); Centro de Investigación Biomédica en Red de Enfermedades Hepáticas y Digestivas (CIBEREHD), 08036 Barcelona (ES)
(72) Inventor: Sánchez Fueyo, Alberto, 08036 Barcelona (ES); Lozano Salvatella, Juan José, 08036 Barcelona (ES); Martínez Llordella, Marc, 08036 Barcelona (ES); Rimola Castella, Antoni, 08036 Barcelona (ES); Bohne, Félix, 08036 Barcelona (DE)
(74) Representative: Illescas, Manuel

(56) References cited:
- WO-A1-2008/081039
- WO-A1-2010/000320
- BOHNE F ET AL: "Transcriptional Profiling of Liver Grafts in Spontaneous Operational Tolerance" TRANSPLANTATION, vol. 90, 1559, 27 July 2010 (2010-07-27), page 13, XP002608445
- MARTÍNEZ-LLORDELLA MARC ET AL: "Using transcriptional profiling to develop a diagnostic test of operational tolerance in liver transplant recipients" JOURNAL OF CLINICAL INVESTIGATION, AMERICAN SOCIETY FOR CLINICAL INVESTIGATION, US LNKD- DOI:10.1172/JCI35342, vol. 118, no. 8, 1 August 2008 (2008-08-01), pages 2845-2857, XP002600788 ISSN: 0021-9738 [retrieved on 2008-07-24]
- MARTÍNEZ-LLORDELLA M ET AL: "Multiparameter immune profiling of operational tolerance in liver transplantation" AMERICAN JOURNAL OF TRANSPLANTATION, BLACKWELL MUNKSGAARD, DK LNKD- DOI:10.1111/J.1600-6143.2006.01621.X, vol. 7, no. 2, 1 February 2007 (2007-02-01), pages 309-319, XP002480488 ISSN: 1600-6135
- KAWASAKI MIKIKO ET AL: "Gene expression profile analysis of the peripheral blood mononuclear cells from tolerant living-donor liver transplant recipients" INTERNATIONAL SURGERY, PICCIN MEDICAL BOOKS, PADOVA, IT, vol. 92, no. 2, 1 January 2007 (2007-01-01), pages 276-286, XP009100254 ISSN: 0020-8868
- BENÍTEZ CARLOS ET AL: "Gene expression profiling and transplantation tolerance in the clinic" TRANSPLANTATION, WILLIAMS AND WILKINS, BALTIMORE US LNKD- DOI:10.1097/TP.0B013E3181AF7D17, vol. 88, no. 3 Suppl, 15 August 2009 (2009-08-15), pages S50-S53, XP009137141 ISSN: 0041-1337
- BARRY R ET AL: "Quantitative protein profiling using antibody arrays" PROTEOMICS, WILEY - VCH VERLAG, WEINHEIM, DE LNKD- DOI:10.1002/PMIC.200300877, vol. 4, no. 12, 1 December 2004 (2004-12-01), pages 3717-3726, XP002545158 ISSN: 1615-9853

## Description

### FIELD OF THE INVENTION

This invention refers to the field of human medicine. More specifically, the present invention is focused on a method for the *in vitro* diagnosis or prognosis of the tolerant state of a patient submitted to liver transplantation, which comprises measuring the expression levels, *in vitro,* in samples of liver tissue, of at least one of the following genes or combinations thereof: *TFRC, CDHR2, HMOX1, MIF, HAMP, IFNG, PEBP1, SLC5A12, ADORA3* and *DAB2.*

### STATE OF THE ART

The long-term survival of transplanted grafts critically depends on the life-long administration of immunosuppressive drugs to prevent graft rejection. These drugs are very effective at preventing graft rejection, but they are also associated with severe side effects, such as nephrotoxicity, an augmented risk of opportunistic infections and tumors, and metabolic complications such as diabetes, hyperlipidemia and arterial hypertension. Due to the side effects of immunosuppressive drugs, the induction of tolerance, defined as a state in which the graft maintains a normal function in the absence of chronic immunosuppression, is one of the main goals of research in transplant immunology. Tolerance induction is possible in a great number of experimental models of transplant in rodents. Nevertheless, the application of these experimental treatments in the clinic has been a failure to a large extent. One of the reasons why clinical application in humans of experimental treatments of tolerance induction has not been successful relates to the lack of an accurate tool to non-invasively diagnose tolerance in human transplant recipients. Recent publications point out the urgent need for this tool (N. Najafian et al 2006 and Newell et al. 2006). On the other hand, maintenance of a normal allograft function despite complete discontinuation of all immunosuppressive drugs is occasionally reported in clinical organ transplantation, particularly following liver transplantation. Patients spontaneously accepting grafts are conventionally considered as "operationally" tolerant, and provide a proof of concept that immunological tolerance can actually be attained in humans. Liver transplantation is the only clinical setting in which tolerance spontaneously occurs in a substantial proportion of patients. Indeed, complete immunosuppression withdrawal can be achieved in around 21% of patients (Lerut, J. et al 2006). Unfortunately, there are currently no means to identify these patients before immunosuppression withdrawal is attempted. For this reason, complete discontinuation of immunosuppressive drugs is rarely attempted in liver transplantation, and thus many patients continue to be unnecessarily immunosuppressed, with the health and economic problems that this involves.

Prior attempts to identify tolerance in transplantation, mainly in kidney and liver recipients, have employed either antigen-specific functional assays or antigen nonspecific tests. In the functional assays recipient T lymphocytes are challenged with donor antigens either *in vitro* or *in vivo* (J. Cai et al 2004), (J. Cai et al 2004) and (E. Jankowska-Gan E et al 2002), (P. Sagoo et al. 2010). These assays are very valuable from a mechanistic point of view, since they are the only tests capable of revealing which pathways are responsible for the specificity of the tolerance state. Unfortunately, these assays are also difficult to perform, highly variable from laboratory to laboratory (difficult to standardize), and require the availability of carefully cryopreserved donor cells. For these reasons, functional assays are not optimal for widespread clinical application, and are currently employed only in selected, highly specialized laboratories, and basically for research purposes.

WO 2010/000320 A1 (INST INVEST S BIOMEDIQUES, 7 January 2010) discloses a method for the in vitro diagnosis or prognosis of the tolerant state of a patient subject of a liver transplantation, comprising measuring the expression levels in a sample of a group of genes and assessing the tolerance or non-tolerance of the patient by comparing the expression level to a reference sample (from a non-tolerant liver transplant recipient).

The antigen-non specific immune monitoring tests constitute a variety of methodologies aiming at the phenotypic characterization of the recipient immune system, without the use of donor antigen challenges. Among these tests, the study of T cell receptor CDR3 length distribution patterns (TcLandscape), peripheral blood cell immunophenotyping by employing flow cytometry, and gene expression profiling have been employed to identify biomarkers characteristic of tolerance in humans. The TcLandscape technique has been employed in peripheral blood to discriminate between tolerant kidney recipients and recipients experiencing chronic rejection (S. Brouard et al. 2005). However, this technique is expensive, is currently only available at one laboratory (Inserm 643 and TcLand Expression in Nantes, France), and has never been validated in liver transplantation. The use of peripheral blood immunophenotyping has been used with peripheral blood samples from both liver and kidney tolerant transplant recipients. At least four studies addressing this methodology are known to inventors. In the first one, from the University of Pittsburgh in USA (G.V. Mazariegos et al 2003), it is said that the ratio between pDC and mDC dendritic cell subsets could discriminate between tolerant and non-tolerant recipients in pediatric liver transplantation. In the second study, from Kyoto (Y. Li et al 2004), it is said that an increased ratio between delta-1 and delta-2 gammadelta T cells in peripheral blood is more prevalent in tolerant than in non-tolerant liver recipients. In the third study, which was coordinated by the inventors (Martinez-Llordella et al 2007), an increased number of CD4+CD25+ T cells and an increased ratio of delta-1 to delta-2 gammadelta T cells were noted in peripheral blood of tolerant liver recipients as compared with non-tolerant recipients. The value of the ratio between delta-1 and delta-2 gammadelta was however questioned in a subsequent study from the same group (Puig-Pey et al. Transplant Int 2010). Furthermore none of these tests offers the accuracy required for a widespread clinical application. The use of gene expression profiling techniques to identify biomarkers of tolerance has been employed both in kidney and in liver transplantation (S.Brouard et al. PNAS 2007; M. Martinez-Llordella et al. J Clin Invest 2008; K.Newell et al. J Clin Invest 2010; P.Sagoo et al. , J Clin Invest 2010). These techniques are easier to standardize than the tests described before. Furthermore, the referenced studies have shown that the use of the identified transcriptional biomarkers is an extremely accurate means to differentiate between tolerant recipients off immunosuppressive drugs and non-tolerant recipients who require maintenance immunosuppression. The main limitation of the studies published in the literature so far is that they have not attempted to prospectively validate their results. In other words, they have not been able to demonstrate whether these biomarkers can identify tolerant recipients before immunosuppression is discontinued. In the absence of this demonstration it is not possible to be certain that the differences observed in gene expression are not actually caused by the effect of pharmacological immunosuppression in the group of non-tolerant recipients. Furthermore, none of the previously reported studies have attempted to investigate whether differences in gene expression also exist at the level of the graft itself.

While the chronic use of immunosuppressive drugs is currently the only means to ensure long-term survival of transplanted allografts, these drugs are expensive and are associated with severe side effects (nephrotoxicity, tumor and infection development, diabetes, cardiovascular complications, etc.) that lead to substantial morbidity and mortality. Hence, any strategy capable of significantly reducing the use of immunosuppressive drugs in transplantation may have a large impact on the health and quality of life of transplant recipients.

In conclusion, the provision of a validated method able to predict tolerance in liver transplant patients, and thus capable of indicating that the administration of immunosuppressive drugs to said patients can be dispensed with, remains being a challenge.

### DESCRIPTION OF THE INVENTION

Therefore, the present invention aims to solve the above cited problem by means of the identification of gene expression profiles as biomarkers, able to predict tolerance in liver transplant patients. Thus, with the objective of identifying those genes showing a statistically significant difference in expression levels between liver recipients who can discontinue immunosuppressive therapy (tolerant) and those who require maintenance immunosuppressive drugs (non-tolerant), biopsy liver tissue samples were collected from a group of stable liver transplant recipients under maintenance immunosuppression therapy who were enrolled in a prospective clinical trial of immunosuppressive drug withdrawal.

The expression profile may be determined by any technology known by a man skilled in the art. In particular, each gene expression level may be measured at the genomic and/or nucleic and/or proteic level. In a preferred embodiment, the expression profile is determined by measuring the amount of nucleic acid transcripts of each gene. In another embodiment, the expression profile is determined by measuring the amount of protein produced by each of the genes.

The amount of nucleic acid transcripts can be measured by any technology known by a man skilled in the art. In particular, the measure may be carried out directly on an extracted messenger RNA (mRNA) sample, or on retrotranscribed complementary DNA (cDNA) prepared from extracted mRNA by technologies well-know in the art. From the mRNA or cDNA sample, the amount of nucleic acid transcripts may be measured using any technology known by a man skilled in the art, including nucleic microarrays, quantitative PCR, and hybridization with a labelled probe.

In a particular embodiment, which should not be considered as limiting the scope of the invention, the determination of the expression profile of these biopsies we first conducted Illumina Beadchip whole-genome expression microarrays, which identified genes with p-value<0.01 and false discovery rate (FDR) < 25% (Table 1).

**Table 1. List of genes differentially expressed between tolerant and non-tolerant liver biopsy samples**

| **Gene** | **NCBI reference sequence (RefSeq)** | **FDR** | **p-value** |
|---|---|---|---|
| *TFRC* | No Annotation | 0 | 1,19E-005 |
| *LOC644037* | XR_017337 | 0 | 2,13E-005 |
| *LOC729266* | XM_001721977 | 10,01418211 | 4,50E-006 |
| *ST7OT1* | NR_002330 | 10,01418211 | 9,12E-006 |
| *MYO19* | NM_001033580 | 10,01418211 | 1,16E-005 |
| *TP5313* | NM_004881 | 10,01418211 | 0,000143629 |
| *HAMP* | NM_021175 | 10,01418211 | 0,001152774 |
| *MCOLN1* | NM_020533 | 15,02127316 | 3,52E-005 |
| *OTUD7A* | NM_130901 | 15,02127316 | 4,21E-005 |
| *EXT2* | NM_000401 | 15,02127316 | 7,92E-005 |
| *KLHL28* | NM_017658 | 15,02127316 | 0,000219206 |
| *UHMK1* | NM_175866 | 15,02127316 | 0,000252044 |
| *FIGF* | NM_004469 | 15,02127316 | 0,000482129 |
| *SLC1A7* | NM_006671 | 15,02127316 | 0,000917616 |
| *ADORA3* | NM_020683 | 15,02127316 | 0,001104349 |
| *SLC5A12* | NM_178498 | 15,02127316 | 0,001594655 |
| *TAF15* | NM_139215 | 15,02127316 | 0,001995782 |
| *TPPP3* | NM_016140 | 16,89893231 | 2,00E-005 |
| *TAGLN* | NM_001001522 | 16,89893231 | 2,71E-005 |
| *NFKBIL2* | NM_013432 | 16,89893231 | 3,36E-005 |
| *OR2C3* | NM_198074 | 16,89893231 | 0,000221256 |
| *UNG* | NM_080911 | 16,89893231 | 0,000297046 |
| *GHSR* | NM_004122 | 16,89893231 | 0,000320149 |
| *KRTAP5-10* | NM_001012710 | 16,89893231 | 0,000411491 |
| *UNC13A* | NM_001080421 | 16,89893231 | 0,000434094 |
| *G3BP1* | NM_198395 | 23,05590765 | 0,000139785 |
| *ANKRD5* | NM_022096 | 23,05590765 | 0,000181261 |
| *RBM23* | NM_018107 | 23,05590765 | 0,000181688 |
| *RAG2* | NM_000536 | 23,05590765 | 0,000191286 |
| *TUBA8* | NM_018943 | 23,05590765 | 0,000201266 |
| *DGKK* | NM_001013742 | 23,05590765 | 0,000210462 |
| *C1orf61* | NM_006365 | 23,05590765 | 0,000286859 |
| *ADSSL1* | NM_199165 | 23,05590765 | 0,0004024 |
| *FBXL4* | NM_012160 | 23,05590765 | 0,000406323 |
| *VAC14* | NM_018052 | 23,05590765 | 0,000426174 |
| *LOC643668* | XR_039201 | 23,05590765 | 0,000427565 |
| *RNASE13* | NM_001012264 | 23,05590765 | 0,000451496 |
| *SAGE1* | NM_018666 | 23,05590765 | 0,000474797 |
| *RTP2* | NM_001004312 | 23,05590765 | 0,000516765 |
| *SYNE2* | NM_182910 | 23,05590765 | 0,00052836 |
| *TSPAN2* | NM_005725 | 23,05590765 | 0,000583719 |
| *SCRG1* | NM_007281 | 23,05590765 | 0,000676665 |
| *ACSL1* | NM_001995 | 23,05590765 | 0,000734359 |
| *STRN4* | NM_013403 | 23,05590765 | 0,000767142 |
| *TUBA4A* | NM_006000 | 23,05590765 | 0,000924509 |
| *RIBC1* | NM_144968 | 23,05590765 | 0,000965443 |
| *MCHR1* | NM_005297 | 23,05590765 | 0,00099149 |
| *MUTED* | NM_201280 | 23,05590765 | 0,001028448 |
| *TANK* | NM_004180 | 23,05590765 | 0,001031594 |
| *DPP4* | NM_001935 | 23,05590765 | 0,001174649 |
| *CHD3* | NM_001005271 | 23,05590765 | 0,00129579 |
| *KLK15* | NM_017509 | 23,05590765 | 0,00133 |
| *NFIX* | NM_002501 | 23,05590765 | 0,001358456 |
| *FAM3B* | NM_206964 | 23,05590765 | 0,001386029 |
| *DOC2A* | NM_003586 | 23,05590765 | 0,001388434 |
| *NQO2* | NM_000904 | 23,05590765 | 0,001470921 |
| *KIAA1143* | NM_020696 | 23,05590765 | 0,001478818 |
| *PLCD3* | NM_133373 | 23,05590765 | 0,001551069 |
| *PLXNA4* | NM_181775 | 23,05590765 | 0,001589304 |
| *ADAMTS3* | NM_014243 | 23,05590765 | 0,001589498 |
| *ABAT* | NM_001127448 | 23,05590765 | 0,001742645 |
| *POF1B* | NM_024921 | 23,05590765 | 0,001746171 |
| *CYP2W1* | NM_017781 | 23,05590765 | 0,001815383 |
| *HSPA1A* | NM_005345 | 23,05590765 | 0,002150872 |
| *FAM162A* | NM_014367 | 23,05590765 | 0,002262613 |
| *KIAA1274* | NM_014431 | 23,05590765 | 0,002458413 |
| *CP* | NM_000096 | 23,05590765 | 0,002664451 |
| *OR5A2* | NM_001001954 | 23,05590765 | 0,002806537 |
| *C9orf127* | NM_001042589 | 23,05590765 | 0,003069775 |
| *AMPD2* | NM_203404 | 23,05590765 | 0,004156098 |
| *KRT19* | NM_002276 | 23,05590765 | 0,006219448 |
| *ATP1B1* | NM_001677 | 23,05590765 | 0,006336133 |
| *C20orf71* | NM_178466 | 24,92892142 | 0,001022188 |
| *LTBP4* | NM_001042544 | 24,92892142 | 0,00131758 |
| *CNTNAP1* | NM_003632 | 24,92892142 | 0,002377442 |
| *FNDC3A* | NM_014923 | 24,92892142 | 0,00241448 |
| *ZNF665* | NM_024733 | 24,92892142 | 0,00332211 |

On the basis of the microarray results and a number of studies conducted in experimental animal models of immunological tolerance, a set of 104 genes (listed in **Table 2**) were then selected for validation employing quantitative real time PCR.

**Table 2. List of genes analysed by real-time PCR**

| **Gene** | **NCBI Gene ID** | **Name** | **Selection criteria** |
|---|---|---|---|
| *18S* | 100008588 | Eukaryotic 18S rRNA | HK |
| *TP5313* | 9540 | tumor protein p53 inducible protein 3 | M |
| *HAMP* | 57817 | hepcidin antimicrobial peptide | M |
| *SAGE1* | 55511 | sarcoma antigen 1 | M |
| *DPP4* | 1803 | dipeptidyl-peptidase 4 | M |
| *MYO19* | 80179 | myosin XIX | M |
| *MCOLN1* | 57192 | mucolipin 1 | M |
| *ACSL1* | 2180 | acyl-CoA synthetase long-chain family member 1 | M |
| *UNG* | 7374 | uracil-DNA glycosylase | M |
| *TFRC* | 7037 | transferrin receptor (p90, CD71) | M |
| *TUBA4A* | 7277 | tubulin, alpha 4a | M |
| *COG5* | 10466 | component of oligomeric golgi complex 5 | M |
| *FAM162A* | 26355 | family with sequence similarity 162, member A | PK |
| *FKBP1A* | 2280 | FK506 binding protein 1A, 12kDa | PK |
| *ABAT* | 18 | 4-aminobutyrate aminotransferase | M |
| *CP* | 1356 | ceruloplasmin (ferroxidase) | PK |
| *HLA-E* | 3133 | major histocompatibility complex, class I, E | PK |
| *CXCR7* | 57007 | chemokine (C-X-C motif) receptor 7 | PK |
| *SRGN* | 5552 | serglycin | PK |
| *PRF1* | 5551 | perforin 1 (pore forming protein) | PK |
| *TLR8* | 51311 | toll-like receptor 8 | PK |
| *STAT1* | 6772 | signal transducer and activator of transcription 1 | PK |
| *IL18BP* | 10068 | interleukin 18 binding protein | PK |
| *PSMB9* | 5698 | proteasome (prosome, macropain) subunit 9 | PK |
| *HFE* | 3077 | hemochromatosis | PK |
| *IRF1* | 3659 | interferon regulatory factor 1 | PK |
| *CXCL9* | 4283 | chemokine (C-X-C motif) ligand 9 | PK |
| *UBD* | 10537 | ubiquitin D | PK |
| *CD8A* | 925 | CD8a molecule | PK |
| *IL32* | 9235 | interleukin 32 | PK |
| *CXCL10* | 3627 | chemokine (C-X-C motif) ligand 10 | PK |
| *CCL3* | 6348 | chemokine (C-C motif) ligand 3 | PK |
| *CD3D* | 915 | CD3d molecule, delta (CD3-TCR complex) | PK |
| *IL6* | 3569 | interleukin 6 (interferon, beta 2) | PK |
| *IL1A* | 3552 | interleukin 1, alpha | PK |
| *IL1B* | 3553 | interleukin 1, beta | PK |
| *TFR2* | 7036 | transferrin receptor 2 | PK |
| *HFE2* | 148738 | hemochromatosis type 2 (juvenile) | PK |
| *BMP4* | 652 | bone morphogenetic protein 4 | PK |
| *SMAD4* | 4089 | SMAD family member 4 | PK |
| *FTH1* | 2495 | ferritin, heavy polypeptide 1 | PK |
| *PDCD1* | 5133 | programmed cell death 1 | PK |
| *HLA-G* | 3135 | major histocompatibility complex, class I, G | PK |
| *FOXP3* | 50943 | forkhead box P3 | PK |
| *IL10* | 3586 | interleukin 10 | PK |
| *TGFB1* | 7040 | transforming growth factor, beta 1 | PK |
| *IL2RB* | 3560 | interleukin 2 receptor, beta | PK |
| *KLRF1* | 51348 | killer cell lectin-like receptor subfamily F, 1 | PK |
| *SLAMF7* | 57823 | SLAM family member 7 | PK |
| *KLRD1* | 3824 | killer cell lectin-like receptor subfamily D, 1 | PK |
| *CX3CR1* | 1524 | chemokine (C-X3-C motif) receptor 1 | PK |
| *LING02* | 158038 | leucine rich repeat and Ig domain containing 2 | PK |
| *BNC2* | 54796 | basonuclin 2 | PK |
| *NCR1* | 9437 | natural cytotoxicity triggering receptor 1 | PK |
| *COL13A1* | 1305 | collagen, type XIII, alpha 1 | PK |
| *IGFBP7* | 3490 | insulin-like growth factor binding protein 7 | PK |
| *SH2D1B* | 117157 | SH2 domain containing 1B | PK |
| *NCAM1* | 4684 | neural cell adhesion molecule 1 | PK |
| *KLRK1* | 22914 | killer cell lectin-like receptor subfamily K, 1 | PK |
| *KLRC1* | 3821 | killer cell lectin-like receptor subfamily C, 1 | PK |
| *MICA* | 4276 | MHC class I polypeptide-related sequence A | PK |
| *MICB* | 4277 | MHC class I polypeptide-related sequence B | PK |
| *TLR4* | 7099 | toll-like receptor 4 | PK |
| *GZMB* | 3002 | granzyme B (granzyme 2) | PK |
| *AP1S2* | 8905 | adaptor-related protein complex 1, sigma 2 | PK |
| *SMARCD3* | 6604 | SWI/SNF related, matrix associated | PK |
| *CD37* | 951 | CD37 molecule | PK |
| *FCER2* | 2208 | Fc fragment of IgE, low affinity II, (CD23) | PK |
| *MS4A1* | 931 | membrane-spanning 4-domains | PK |
| *CXCR3* | 2833 | chemokine (C-X-C motif) receptor 3 | PK |
| *CXCL11* | 6373 | chemokine (C-X-C motif) ligand 11 | PK |
| *IFNG* | 3458 | interferon, gamma | PK |
| *CD274* | 29126 | CD274 molecule | PK |
| *PDCD1LG2* | 80380 | programmed cell death 1 ligand 2 | PK |
| *C3* | 718 | complement component 3 | PK |
| *TBX21* | 30009 | T-box 21 | PK |
| *GATA3* | 2625 | GATA binding protein 3 | PK |
| *FAS* | 355 | Fas (TNF receptor superfamily, member 6) | PK |
| *FASLG* | 356 | Fas ligand (TNF superfamily, member 6) | PK |
| *RORC* | 6097 | RAR-related orphan receptor C | PK |
| *HMOX1* | 3162 | heme oxygenase (decycling) 1 | PK |
| *TNFAIP3* | 7128 | tumor necrosis factor, alpha-induced protein 3 | PK |
| *BCL2* | 596 | B-cell CLL/lymphoma 2 | PK |
| *SOCS1* | 8651 | suppressor of cytokine signaling 1 | PK |
| *TNF* | 7124 | tumor necrosis factor (TNF superfamily, 2) | PK |
| *NOS2* | 4843 | nitric oxide synthase 2, inducible | PK |
| *IL12B* | 3593 | interleukin 12B (natural killer cell stimulatory 2) | PK |
| *IL18* | 3606 | interleukin 18 (interferon-gamma-inducing factor) | PK |
| *IRF3* | 3661 | interferon regulatory factor 3 | PK |
| *CCL21* | 6366 | chemokine (C-C motif) ligand 21 | PK |
| *HPRT1* | 3251 | hypoxanthine phosphoribosyltransferase 1 | HK |
| *GAPDH* | 2597 | glyceraldehyde-3-phosphate dehydrogenase | HK |
| *DTWD2* | 285605 | DTW domain containing 2 | M |
| *POF1B* | 79983 | premature ovarian failure, 1B | M |
| *MYD88* | 4615 | myeloid differentiation primary response gene (88) | PK |
| *DAB2* | 1601 | disabled homolog 2 | M |
| *TIPARP* | 25976 | TCDD-inducible poly(ADP-ribose) polymerase | M |
| *RBM23* | 55147 | RNA binding motif protein 23 | M |
| *TTC3* | 7267 | tetratricopeptide repeat domain 3 | M |
| *MIF* | 4282 | macrophage migration inhibitory factor | M |
| *PEBP1* | 5037 | phosphatidylethanolamine binding protein 1 | M |
| *SLC5A12* | 159963 | solute carrier family 5 member 12 | M |
| *FABP4* | 2167 | fatty acid binding protein 4 | M |
| *PCDH24* | 54825 | protocadherin 24 | M |
| *VNN3* | 55350 | vanin 3 | M |
| *ADORA3* | 140 | adenosine A3 receptor | M |
| *TAF15* | 8148 | TATA box binding protein (TBP)-associated factor | M |

| | | | |
|---|---|---|---|
| **(M=significant in microarray, PK=previous knowledge, HK=houskeeping control)** **NCBI accession date: 28^{th} July 2010** | | | |

The results of the experiments conducted by real-time PCR revealed that the genes listed in **Table 3** show a statistically significant difference in expression between biopsies taken from liver transplant patients who can safely abandon immunosuppressive drugs (tolerant) and patients who undergo rejection when immunosuppressive drugs are discontinued (non-tolerant). As shown in **Table 3** the genes *TFRC* and *MIF* are up-regulated, and the genes *CDHR2, HMOX1, HAMP, IFNG, PEBP1, SLC5A12, ADORA3* and *DAB2* are down-regulated, in tolerant liver transplant recipients as compared with a reference RNA sample (which can be a pool of RNAs obtained from healthy non-transplanted liver tissue, a reference RNA such as the commercially available Human Liver Total RNA from Ambion, or an absolute reference consisting in a sample containing a previously quantified number of RNA molecules).

**Table 3**

| Gene | p-value | | |
|---|---|---|---|
| | Student t | Wilcox | fold change |
| TFRC | 0,000035 | 0,000026 | -2,505329 |
| CDHR2 | 0,006059 | 0,004665 | 1,747146 |
| HMOX1 | 0,007195 | 0,005044 | 1,399586 |
| MIF | 0,008793 | 0,003526 | -1,547565 |
| HAMP | 0,012583 | 0,077430 | 2,173470 |
| IFNG | 0,013215 | 0,020816 | 1,319508 |
| PEBP1 | 0,023371 | 0,011855 | 1,132884 |
| SLC5A12 | 0,032834 | 0,022216 | 2,345670 |
| ADORA3 | 0,039721 | 0,041890 | 1,464086 |
| DAB2 | 0,046314 | 0,049169 | 1,193336 |

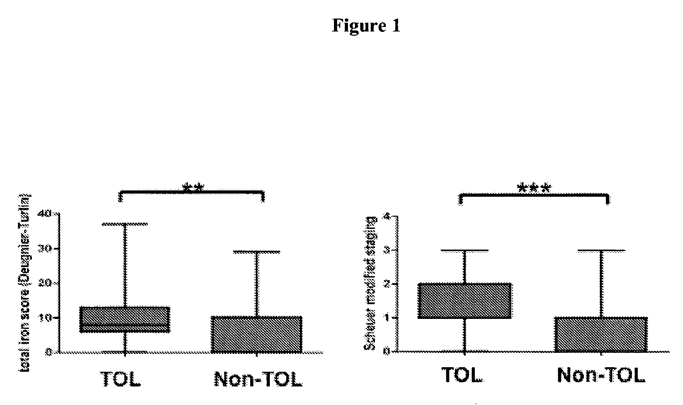

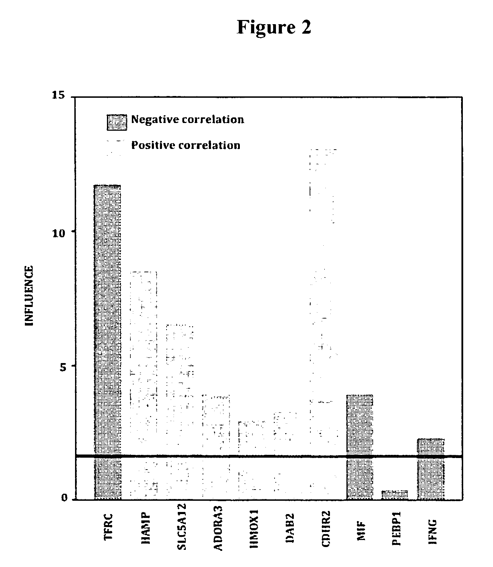

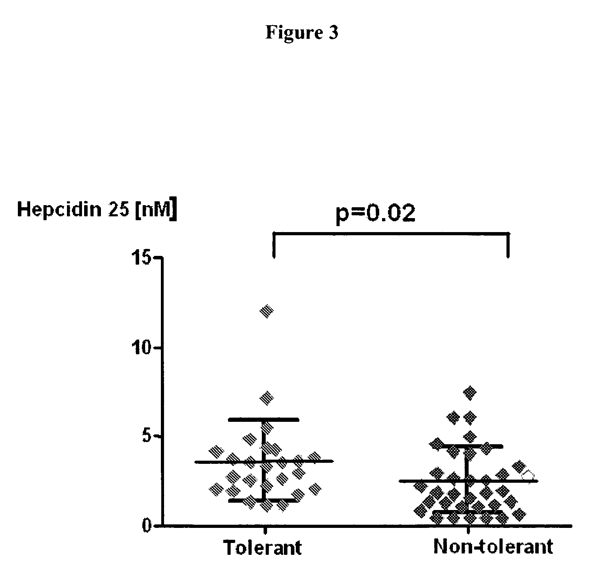

It is important to note that the genes comprised in **Table 3** share a functional pathway because most of them are involved in the regulation of iron metabolism. In fact, the biopsies of patients who can successfully discontinue the immunosuppressive medication showed a greater accumulation of iron, as shown in **Figure 1****.** It is known that the genes *TFRC, HAMP, IFNG* and *HMOX1* are directly involved in the control of cellular iron metabolism. Moreover, the expression of many of the genes differentially expressed between patients in whom it is possible to remove the immunosuppressive medication (tolerant) and those in whom said removal is not advisable (non- tolerant), such *CDHR2, MIF, SLC5A12, ADORA3* and *DAB2,* significantly correlates with the deposition of intra-hepatic iron (measured by the modified method of Scheuer or the *total iron score* method; see **Figure 2**). An additional evidence emphasizing the role of iron metabolism in the acquisition of operational tolerance to liver allografts is the observation that the serum levels of hepcidin (the most important hormone in the regulation of systemic iron homeostasis, which is encoded by *HAMP*) are significantly higher in tolerant than in non-tolerant liver recipients (see **Figure 3**). Taken together, these results indicate that the regulation of iron metabolism at the intrahepatic level plays a role in the control of allo-immune responses in liver transplantation.

More specifically, these results indicate that the expression level of the genes involved in said regulation of iron metabolism should be particularly relevant for the design of a method according to the present disclosure.

Thus, the first embodiment of the present invention refers to a method for the *in vitro* diagnosis or prognosis of the tolerant state of a patient subjected to a liver transplantation that comprises:
a. Measuring, in a biological sample obtained from the liver allograft of the patient under investigation, the expression level of at least one of the following genes or combinations thereof: *TFRC*, *CDHR2*, *HMOX1*, *MIF*, *HAMP, IFNG, PEBP1, SLC5A12*, *ADORA3* and *DAB2*;
b. Assessing the tolerance or non-tolerance of the patient under investigation to a liver transplantation by comparing the expression level of at least one of the genes or combinations thereof, of the step a), with the expression level of the same genes or combinations thereof, taken from a reference sample.

The reference sample is a predetermined expression profile, obtained from a biological sample from a subject obtained from healthy non-transplanted liver tissue. It can be a pool of RNAs, a reference RNA such as the commercially available Human Liver Total RNA from Ambion, or an absolute reference consisting in a sample containing a previously quantified number of RNA molecules).

As shown in **Table 4** below, measurement of the expression level of each of the genes comprised in **Table 3** is useful for the identification of patients who can safely discontinue all immunosuppressive medication without undergoing rejection (tolerance). Therefore, this **Table 4** shows the capacity of the individual genes listed therein to statistically differentiate the patients who will tolerate the transplanted liver in the absence of immunosuppressive therapy, from those recipients who will reject when immunosuppressive medications are discontinued.

**Table 4**

| **GENES** | **AUC** | **SN** | **SP** | **ER** | **PPV** | **NPV** |
|---|---|---|---|---|---|---|
| *TFRC* | *0,76* | *51,72* | *90,48* | *25,35* | *78,95* | *73, 08* |
| *CDHR2* | *0,70* | *58, 62* | *83,33* | *26,76* | *70,83* | *74,47* |
| *HMOX1* | *0,70* | *55,17* | *83,33* | *28,17* | *69,57* | *72,92* |
| *PEBP1* | *0,68* | *44,83* | *90,48* | *28,17* | *76,47* | *70,37* |
| *MIF* | *0,68* | *93,1* | *52,38* | *30,99* | *57,45* | *91, 67* |
| *SLC5A12* | *0,66* | *44,83* | *85,71* | *30,99* | *68,42* | *69,23* |
| *DAB2* | *0,65* | *58,62* | *73,81* | *32,39* | *60,71* | *72,09* |
| *IFNG* | *0,64* | *31,03* | *90,48* | *33,8* | *69,23* | *65,52* |
| *HAMP* | *0,63* | *86,21* | *52,38* | *33,8* | *55,56* | *84,62* |
| *ADORA3* | *0,65* | *20,69* | *95,24* | *35,21* | *75* | *63,49* |

| | | | | | | |
|---|---|---|---|---|---|---|
| AUC: area under the curve SN: sensitivity SP: specificity ER: error rate PPV: positive predictive value NPV: negative predictive value | | | | | | |

However, although the genes cited in **Tables 3** or **4** have an individual predictive capacity, different clusters were made departing from some combinations of said genes, with the aim of identifying a predictive method as accurate as possible. Moreover, the genes listed in **Tables 3** or **4** were also grouped with other genes which did not show a predictive value *per se* (as taken independently), for example: *LC5A12, VNN3, SOCS1, TTC3, RBM23, SH2D1B, NCR1, TFRC, TUBA4A, TAF15, TIPARP, MOX1, MCOLN1, EBP1, DHR2,* and *AB2.* Therefore, in a preferred embodiment, the present invention further comprises measuring the expression levels of at least one of the following genes: *LC5A12, VNN3, SOCS1, TTC3, RBM23, SH2D1B, NCR1, TFRC, TUBA4A, TAF15, TIPARP, MOX1, MCOLN1, EBP1, DHR2, and AB2* in combination with at least one of the genes listed in **Table 3** or **4.**

In order to identify the combination/s of gene expression biomarkers with the best performance in the diagnosis of the outcome of immunosuppression drug withdrawal in liver transplantation, we conducted an exhaustive search for predictive models employing the linear discriminant analysis and logistic regression algorithms implemented in the *misclassification penalized posterior* (MiPP) software. First, we conducted a 10-fold cross-validation step on a group of liver samples (18 tolerant and 31 non-tolerant) collected from patients enrolled in Hospital Clinic Barcelona. Next, random splitting cross-validation of the diagnosis models was conducted on the whole data set (which included the 56 samples from Barcelona and 21 additional samples from Rome and Leuven) by repeatedly partitioning it into training set (2/3) and independent test set (1/3) for external model validation. In addition, for each model identified in the training set the optimal probability cut-off of tolerance was computed employing ROC (Receiver Operating Curves) analysis. To demonstrate that the performance of the models was not center-dependent, we then computed SN, SP, NPV, PPV and overall error rates for the samples collected from Barcelona and those obtained from Rome and Leuven. Importantly, all gene expression measurements were performed on samples obtained before immunosuppression medications were discontinued. Our results therefore indicate that the identified genetic markers are capable of predicting the success of immunosuppression drug withdrawal.

As cited above, this type of analysis takes into account not only those genes found to be differently expressed genes (**Table 3**) but also genes that, although they are not statistically differentially expressed, as taken independently, they do contribute to optimize the diagnosis in combination with the genes of **Table 3. Table 5** shows the groups of samples employed for the design and evaluation of the predictive models based on the expression in liver biopsy of the genes measured by real-time PCR. Importantly, while the samples collected from Barcelona recipients were employed for both microarray and qPCR experiments, none of the samples obtained from Rome and Leuven were employed in the microarray experiments.

**Table 5**

| | **TOL (n)** | **Non-TOL (n)** | **origin** |
|---|---|---|---|
| **Training set** | **18** | **31** | **Barcelona** |
| **Test set** | **10** | **11** | **Rome/Leuven** |

Thus, **Table 6** shows the combinations of genes whose expression best classifies patients into the non-tolerant or tolerant categories according to the results of the qPCR expression measurements. A classification error of less than 15% in the learning group and less than 10% in the validation group was arbitrary selected to select the most accurate and clinically useful models.

**Table 6**

| | | **Barcelona** | | | **Rome + Leuven** | | |
|---|---|---|---|---|---|---|---|
| **Genes** | **n** | **SN** | **SP** | **ER** | **SN** | **SP** | **ER** |
| *SLC5A12*+*VNN3*+*TFRC*+*SOCS1*+*MIF*+*TTC3*+*RB M23*+*PEBP1*+*SH2D1B*+*NCR1*+*DAB2*+*ADORA3* | 12 | 100 | 90,32 | 6,12 | 90 | 90,91 | 9,52 |
| *TFRC*+*PEBP1*+*MIF*+*CDHR2*+*HAMP*+*TUBA4A*+*TT C3*+*HMOX1*+*VNN3*+*NCR1*+*ADORA3*+*TAF15*+*IFN G*+*SOCS1*+*TIPARP* | 15 | 94,44 | 93,55 | 6,12 | 80 | 100 | 9,52 |
| *HMOX1*+*CDHR2*+*MIF*+*PEBP1*+*TFRC*+*SLC5A12*+ *SOCS1*+*HAMP*+*VNN3*+*IFNG* | 10 | 94,44 | 90,32 | 8,16 | 80 | 100 | 9,52 |
| *TFRC*+*PEBP1*+*MIF*+*CDHR2*+*SLC5A12*+*HAMP*+*S OCS1*+*IFNG*+*HMOX1* | 9 | 94,44 | 90,32 | 8,16 | 80 | 100 | 9,52 |
| *TFRC*+*IFNG*+*CDHR2*+*ADORA3*+*HAMP*+*MIF*+*PEB P1*+*VNN3*+*SOCS1*+*HMOX1*+*DAB2* | 11 | 88,89 | 90,32 | 10,2 | 80 | 100 | 9,52 |
| *TFRC*+*DAB2*+*MIF*+*PEBP1*+*IFNG*+*HAMP*+*SLC5A1 2*+*SOCS1*+*VNN3*+*ADORA3*+*CDHR2*+*MCOLN1*+*H MOX1* | 13 | 77,78 | 96,77 | 10,2 | 80 | 100 | 9,52 |
| *TFRC*+*IFNG*+*HMOX1*+*MCOLN1*+*MIF*+*HAMP*+*AD ORA3*+*CDHR2*+*PEBP1*+*SOCS1* | 10 | 88,89 | 90,32 | 10,2 | 80 | 100 | 9,52 |
| *PEBP1*+*TFRC*+*HMOX1*+*IFNG*+*MCOLN1*+*SOCS1* +*MIF*+*CDHR2*+*HAMP*+*ADORA3* | 10 | 88,89 | 90,32 | 10,2 | 80 | 100 | 9,52 |
| *TFRC*+*PEBP1*+*IFNG*+*CDHR2*+*ADORA3*+*VNN3*+*H MOX1*+*DA82*+*SOCS1*+*MIF*+*HAMP* | 11 | 88,89 | 90,32 | 10,2 | 80 | 100 | 9,52 |
| *CDHR2*+*ADORA3*+*IFNG*+*TFRC*+*VNN3*+*HMOX1*+ *PEBP1*+*MIF*+*SLC5A12*+*HAMP*+*SOCS1*+*MCOLN1* | 12 | 77,78 | 96,77 | 10,2 | 80 | 100 | 9,52 |
| *SLCSA12*+*TFRC*+*IFNG*+*MIF*+*DAB2*+*HMOX1*+*CD HR2*+*SOCS1*+*HAMP*+*PEBP1*+*VNN3*+*ADORA3*+*M COLN1* | 13 | 77,78 | 96,77 | 10,2 | 80 | 100 | 9,52 |
| *TFRC*+*SOCS1*+*HMOX1*+*PEBP1*+*VNN3*+*CDHR2*+ *HAMP*+*IFNG*+*DAB2*+*MCOLN1*+*ADORA3*+*MIF* | 12 | 83,33 | 93,55 | 10,2 | 80 | 100 | 9,52 |
| *TFRC*+*PEBP1*+*VNN3*+*SOCS1*+*MIF*+*HMOX1*+*DAB 2*+*HAMP*+*IFNG*+*CDHR2*+*ADORA3*+*MCOLN1* | 12 | 83,33 | 93,55 | 10,2 | 80 | 100 | 9,52 |
| *SLCSA12*+*MIF*+*CDHR2*+*TFRC*+*IFNG*+*ADORA3*+ *HAMP*+*VNN3*+*SOCS1*+*MCOLN1*+*PEBP1*+*HMOX1* | 12 | 77,78 | 96,77 | 10,2 | 80 | 100 | 9,52 |
| *TFRC*+*IFNG*+*CDHR2*+*ADORA3*+*PEBP1*+*VNN3*+ *MIF*+*HMOX1*+*MCOLN1*+*SOCS1*+*SLCSA12*+*DA82* ,+*HAMP* | 13 | 77,78 | 96,77 | 10,2 | 80 | 100 | 9,52 |
| *TFRC*+*VNN3*+*HAMP*+*CDHR2*+*SLCSA12*+*HMOX1* +*SOCS1*+*PEBP1*+*MIF* | 9 | 94,44 | 83,87 | 12,24 | 80 | 100 | 9,52 |
| *DAB2*+*TFRC*+*MIF*+*CDHR2*+*PEBP1*+*VNN3*+*TTC3* +*HMOX1*+*SOCS1* | 9 | 83,33 | 90,32 | 12,24 | 80 | 100 | 9,52 |
| *TFRC*+*PEBP1*+*MIF*+*CDHR2*+*VNN3*+*IFNG*+*MCOL N1*+*SOCS1* | 8 | 88,89 | 83,87 | 14,29 | 80 | 100 | 9,52 |
| *TFRC*+*PEBP1*+*MIF*+*SOCS1*+*CDHR2* | 5 | 94,44 | 80,65 | 14,29 | 80 | 100 | 9,52 |

| | | | | | | | |
|---|---|---|---|---|---|---|---|
| SN: sensitivity SP: specificity ER: error rate PPV: positive predictive value NPV: negative predictive value | | | | | | | |

One embodiment of present invention refers to use of at least one of the following genes or combinations thereof: *TFRC, CDHR2, HMOX1, MIF, HAMP, IFNG, PEBP1, SLC5A12, ADORA3* and *DAB2,* in a method for the *in vitro* diagnosis or prognosis of the tolerant state of a patient subjected to a liver transplantation. In a preferred embodiment the method of the invention is carried out using a combination of at least one of the above cited genes with at least one of the following genes: *LCSA12, VNN3, SOCS1, TTC3, RBM23, SH2D1B, NCR1, TFRC, TUBA4A, TAF15, TIPARP, MOX1, MCOLN1, EBP1, DHR2,* and *AB2.* In a particularly preferred embodiment the method of the invention is carried using one of the following gene combinations: *LCSA12, VNN3, TFRC, SOCS1, MIF, TTC3, RBM23, PEBP1, SH2D1B, NCR1, DAB2* and *ADORA3; TFRC, PEBP1, MIF, CDHR2, HAMP, TUBA4A, TTC3, HMOX1, VNN3, NCR1, ADORA3, TAF15, IFNG, SOCS1* and *TIPARP; MOX1, CDHR2, MIF, PEBP1, TFRC, SLC5A12, SOCS1, HAMP, VNN3* and *IFNG; TFRC, PEBP1, MIF, CDHR2, SLC5A12, HAMP, SOCS1, IFNG* and *HMOX1; TFRC, IFNG, CDHR2, ADORA3, HAMP, MIF, PEBP1, VNN3, SOCS1, HMOX1* and *DAB2; TFRC, DAB2, MIF, PEBP1, IFNG, HAMP, SLC5A12, SOCS1, VNN3, ADORA3, CDHR2, MCOLN1* and *HMOX1; TFRC, IFNG, HMOX1, MCOLN1, MIF, HAMP, ADORA3, CDHR2, PEBP1* and *SOCS1; EBP1, TFRC, HMOX1, IFNG, MCOLN1, SOCS1, MIF, CDHR2, HAMP* and *ADORA3; TFRC, PEBP1*, *IFNG, CDHR2, ADORA3, VNN3, HMOX1, DAB2, SOCS1, MIF* and *HAMP; DHR2, ADORA3, IFNG, TFRC, YNN3, HMOX1, PEBP1, MIF, SLCSA12, HAMP, SOCS1* and *MCOLN1; LCSA12, TFRC, IFNG, MIF, DAB2, HMOX1, CDHR2, SOCS1, HAMP, PEBP1, VNN3, ADORA3* and *MCOLN1; TFRC, SOCS1, HMOX1, PEBP1, VNN3, CDHR2, HAMP, IFNG, DAB2, MCOLN1, ADORA3* and *MIF_{;} TFRC, PEBP1, VNN3, SOCS1, MIF, HMOX1, DAB2, HAMP, IFNG, CDHR2, ADORA3* and *MCOLN1; LC5A12, MIF, CDHR2, TFRC, IFNG, ADORA3, HAMP, VNN3, SOCS1, MCOLN1, PEBP1* and *HMOX1; TFRC, IFNG, CDHR2, ADORA3, PEBP1, VNN3, MIF, HMOX1, MCOLN1, SOCS1, SLC5A12, DAB2* and *HAMP; TFRC, VNN3, HAMP, CDHR2, SLC5A12, HMOX1, SOCS1, PEBP1* and *MIF; AB2, TFRC, MIF, CDHR2, PEBP1, VNN3, TTC3, HMOX1* and *SOCS1; TFRC, PEBP1, MIF, CDHR2, VNN3, IFNG, MCOLN1* and *SOC S1; TFRC, PEBP1, MIF, SOCS1* and *CDHR2.*

Some clinical variables may influence the development of operational tolerance following liver transplantation in humans. In particular, the recipients who have been transplanted for a longer period of time or who are older have a higher likelihood of being capable of successfully discontinuing immunosuppressive medications. We conducted additional logistic regression analyses to exclude the potentially confounding effect of these 2 clinical variables on the gene expression measurements. Out of the genes depicted in Tables 3 or 4, the following genes were found to be statistically significant after excluding the effect of recipient age and time since transplantation (Table 7).

**Table 7**

| **Genes** | **p-value** |
|---|---|
| *TFRC* | 0,0033 |
| *PEBP1* | 0,0125 |
| *HMOX1* | 0,0177 |
| *IFNG* | 0,0198 |
| *CDHR2* | 0,0234 |
| *DAB2* | 0,0572 |

In order to develop a genetic predictor independent from clinical variables, we employed logistic regression and the MiPP software on the genes shown in Table 7. The following model was found to be the best predictor in both the learning and the validation groups of patients (Table 8).

**Table 8**

| | | **Barcelona** | | | **Rome+Leuven** | | |
|---|---|---|---|---|---|---|---|
| **Genes** | **n** | **SN** | **SP** | **ER** | **SN** | **SP** | **ER** |
| *TFRC + IFNG + CDHR2* | 3 | 83.3 | 83.9 | 16.3 | 60 | 100 | 19 |

Thus, an additional embodiment of the present invention refers to a method for the *in vitro* diagnosis or prognosis of the tolerant state of a patient subjected to a liver transplantation by measuring gene expression of the following combination of genes: *TFRC, IFNG* and *CDHR2*,.

In a particular embodiment of a method according to the invention, said method may further comprise determining at least one additional parameter useful for the diagnosis or prognosis. Such "parameters useful for the diagnosis" are parameters that cannot be used alone for a diagnosis but that have been described as displaying significantly different values between tolerant subjects and subjects who clearly need immunosuppressive treatment and may thus also be used to refine and/or confirm the diagnosis according to the above described method according to the invention. They may notably include the measurement of the serum levels of proteins encoded by the genes involved in the regulation of the iron metabolism in liver, such as Hepcidin.

A further embodiment of the invention is a method such as described above and which further comprises the determination of the age of the patient and/or the post-transplantation time.

Another embodiment disclosed refers to a kit, for performing the method of the invention for the *in vitro* diagnosis or prognosis of the tolerant state of a patient subjected to a liver transplantation, comprising (i) means for measuring the gene expression levels of the corresponding genes, and (ii) instructions for correlating said gene expression levels above or below the expression level of the same genes taken from a reference RNA sample.

Said reference samples can be a pool of RNAs obtained from healthy non-transplanted liver tissue, a reference RNA such as the commercially available Human Liver Total RNA from Ambion, or an absolute reference consisting in a sample containing a previously quantified number of RNA molecules). In a preferred embodiment the means comprise a microarray or a gene chip which comprises nucleic acid probes, said nucleic acid probes comprising sequences that specifically hybridize to the transcripts of the corresponding set of genes, along with reagents for performing a microarray analysis. In another preferred embodiment of the invention the kit comprises oligonucleotide primers (i.e. *HS02559818s1* for gene *TFRC* or *Hs01125168m1* for gene VNN3), for performing a quantitative reverse transcription polymerase chain reaction, said primers comprising sequences that specifically hybridize to the complementary DNA derived from the transcripts of the corresponding set of genes. Moreover the kit of the invention may comprise a solid support wherein nucleic acid probes which comprises sequences that specifically hybridize to the transcripts of the corresponding set of genes, are displayed thereon.

In another embodiment, the means comprises a microrarray or a protein chip which comprises specific binding moieties such as monoclonal antibodies or fragments thereof.

In one embodiment the kit measures the expression of at least one of the following genes or combinations thereof: *TFRC, CDHR2, HMOX1, MIF, HAMP, IFNG, PEBP1, SLC5A12, ADORA3* and *DAB2,* for the *in vitro* diagnosis or prognosis of the tolerant state of a patient subjected to a liver transplantation. In a preferred embodiment the kit measures the gene expression of a combination of at least one of the above cited genes with at least one of the following genes: *LC5A12, VNN3, SOCS1, TTC3, RBM23, SH2D1B, NCR1, TFRC, TUBA4A, TAF15, TIPARP, MOX1, MCOLN1, EBP1, DHR2,* and *AB2.* In a particularly preferred embodiment the kit measures gene expression of the following gene combinations: *LC5A12, VNN3, TFRC, SOCS1, MIF, TTC3, RBM23, PEBP1, SH2D1B, NCR1, DAB2 andADORA3; TFRC, PEBP1, MIF, CDHR2, HAMP, TUBA4A, TTC3, HMOX1, VNN3, NCR1, ADORA3, TAF15, IFNG, SOCS1* and *TIPARP; MOX1, CDHR2, MIF, PEBP1, TFRC, SLC5A12, SOCS1, HAMP, VNN3* and *IFNG; TFRC, PEEP1, MIF, CDHR2, SLC5A12, HAMP, SOCS1, IFNG* and *HMOX1; TFRC, IFNG, CDHR2, ADORA3, HAMP, MIF, PEBP1*, *VNN3, SOCS1, HMOX1* and *DAB2; TFRC, DAB2, MIF, PEBP1, IFNG, HAMP, SLC5A12, SOCS1, VNN3, ADORA3, CDHR2, MCOLN1* and *HMOX1; TFRC, IFNG, HMOX1, MCOLN1, MIF, HAMP, ADORA3, CDHR2, PEBP1* and *SOCS1; EBP1, TFRC, HMOX1, IFNG, MCOLN1, SOCS1, MIF, CDHR2, HAMP* and *ADORA3; TFRC, PEBP1*, *IFNG, CDHR2, ADORA3, VNN3, HMOX1, DAB2, SOCS1, MIF* and *HAMP; DHR2, ADORA3, IFNG, TFRC, VNN3, HMOX1, PEBP1, MIF, SLC5A12, HAMP, SOCS1* and *MCOLN1; LC5A12, TFRC, IFNG, MIF, DAB2, HMOX1, CDHR2, SOCS1, HAMP, PEBP1, VNN3, ADORA3* and *MCOLN1; TFRC, SOCS1, HMOX1, PEBP1, VNN3, CDHR2, HAMP, IFNG, DAB2, MCOLN1, ADORA3* and *MIF; TFRC, PEBP1, VNN3, SOCS1, MIF, HMOX1, DAB2, HAMP, IFNG, CDHR2, ADORA3* and *MCOLN1; LC5A12, MIF, CDHR2, TFRC, IFNG, ADORA3, HAMP, VNN3, SOCS1, MCOLN1, PEBP1* and *HMOX1; TFRC, IFNG, CDHR2, ADORA3, PEBP1, VNN3, MIF, HMOX1, MCOLN1, SOCS1, SLCSA12, DAB2* and *HAMP; TFRC, VNN3, HAMP, CDHR2, SLCSA12, HMOX1, SOCS1, PEBP1* and *MIF; AB2, TFRC, MIF, CDHR2, PEBP1, VNN3, TTC3, HMOX1* and *SOCS1; TFRC, PEBP1, MIF, CDHR2, VNN3, IFNG, MCOLN1* and *SOC S1; TFRC, PEBP1, MIF, SOCS1* and *CDHR2.*

One of the preferred embodiments refers to a kit for the *in vitro* diagnosis or prognosis of the tolerant state of a patient subjected to a liver transplantation which measures gene expression of the following combination of genes: *TFRC, IFNG* and *CDHR2,.*

Kits, according to present disclosure may further comprise reagents for performing a microarray analysis and/or solid supports wherein nucleic acid probes which comprises sequences that specifically hybridize to the transcripts of the corresponding set of genes, are displayed thereon.

Another embodiment refers to a kit for selecting or modifying an immunotherapy treatment protocol by assessing the tolerant state of the liver recipient by using the above disclosed method or kit.

The last embodiment refers to a method for adapting the immunosuppressive treatment of a liver grafted patient, said method comprising the use of above disclosed method and kits.

The state of the art comprises (Benitez C et al., Abstract #517, American Transplant Congress, San Diego, CA, May 3 - May 5, 2010) the measure in peripheral blood samples of the expression of a group of genes *(KLRF1, PTGDR, NCALD, CD160, IL2RB, PTCH1, ERBB2, KLRB1, NKG7, KLRD1, FEZ1, GNPTAB, SLAMF7, CLIC3, CX3CR1, WDR67, MAN1A1, CD9, FLJ14213, FEMIC, CD244, PSMD14, CTBP2, ZNF295, ZNF267, RGS3, PDE4B, ALG8, GEMIN7)* different from that presented in the present invention, as a method to identify tolerant liver recipients. A comparative assay was carried out (see **Example 10**) in order to determine whether the genes which form part of the present invention have a higher discriminative power as compared with the previously disclosed genes in peripheral blood. It was concluded the measurement of the expression of the genes comprised in the present invention in liver tissue samples, appears as least as accurate than the measurement of the genes comprised in the state of the art in peripheral blood, in order to identify the liver recipients who can successfully leave the immunosuppressive medication because they are tolerant to the transplantation.

### Brief description of the figures

**Figure 1****.** The liver iron content (measured in a semi-quantitative manner after Perls' staining employing either the Scheuer modified method or the *total iron score* method) is higher in the livers of patients who successfully leave the immunosuppressive therapy (TOL) than in those where this is not possible (Non-TOL).
**Figure 2****.** This figure shows that the deposition of intra-hepatic iron (measured by the modified method of Scheuer) correlates with the expression of many of the genes differentially expressed between patients in whom it is possible to discontinue the immunosuppressive medication (tolerant) and those it is not possible (non- tolerant).
**Figure 3****.** This figure shows that the serum levels of hepcidin (the peptide encoded by the gene *HAMP)* are increased in tolerant recipients as compared with non-tolerant liver recipients.

### EXAMPLES

### Example 1. Patient population and study design.

Blood and liver biopsy specimens were collected from a group of liver transplant recipients enrolled in a prospective European Commission supported multi-center clinical trial of immunosuppressive drug withdrawal in liver transplantation (Title: *Search for the immunological Signature of Operational Tolerance in Liver Transplantation;* clinicaltrials.gov identification NCT00647283). Inclusion criteria were the following: 1) >3 years after transplantation; 2) stable liver function and no episodes of rejection during the 12 months prior to inclusion; 3) no history of autoimmune liver disease; 4) pre-inclusion liver biopsy without significant abnormalities (no signs of acute or chronic rejection according to Banff criteria; absence of portal inflammation in >50% of portal tracts; absence of central perivenulitits in >50% of central veins; absence of bridging fibrosis or cirrhosis). In enrolled recipients immunosuppressive drugs were gradually weaned until complete discontinuation over a 6-9 month period and then followed-up for 12 additional months. Patients were considered as tolerant if no rejection episodes occurred during the entire duration of the study and no significant histological changes were noted in a liver biopsy obtained at the end of the 12-month follow-up period. Patients undergoing acute rejection during the study were considered as non-tolerant. Out of the 102 recipients enrolled in the trial 79 (33 tolerant and 46 non-tolerant) were included in the current study. Blood and liver biopsy specimens available for the study were obtained before immunosuppressive drugs were discontinued from both tolerant (**TOL,** n=33) and non-tolerant (**Non-TOL,** n=46) recipients, at the time of rejection from non-tolerant recipients (n=14), and at the end of the study in tolerant recipients (n=4). In addition, liver tissue samples were also obtained from the following patient groups: a) liver transplant recipients with chronic hepatitis due to recurrent hepatitis C virus infection (**HEPC,** n=12); b) liver transplant recipients with typical acute cellular rejection taking place during the immediate post-transplant period (**REJ,** n=9); c) liver transplant recipients under maintenance immunosuppression with normal liver function and normal liver histology 1 year after transplantation (**CONT-Tx,** n=8); and d) non-transplanted patients undergoing surgery for colorectal liver metastases (**CONT,** n=10). Participating recipients were enrolled from Hospital Clinic Barcelona (Spain), University Tor Vergata Rome (Italy) and University Hospitals Leuven (Belgium). The study was approved by the institutional review boards of the three participating institutions and written informed consent was obtained from all study patients. Clinical and demographic characteristics of patients included in the study are summarized in Table 1 and an outline of the study design is depicted in Figure 1. A detailed description of the patient population and clinical outcomes of the immunosuppression withdrawal clinical trial will be reported elsewhere.

### Example 2. Liver biopsy specimens and histological assessment.

Liver biopsies were performed percutaneously under local anaesthesia. A 2-3 mm portion of the needle biopsy liver cylinder was immediately preserved in RNAlater reagent (Ambion, Austin, USA), kept at 4°C for 24h and then cryopreserved in liquid nitrogen after removal of the RNAlater reagent. The remaining cylinder was fromalin- fixed and paraffin-embedded. In CONT patients surgical liver biopsies of non-tumoral livers were obtained and processed as previously described. For histological assessment 3 µm thick slides were stained using hematoxylin-eosin. Masson's trichrome for connective tissue analysis and Perl's for iron content assessment. The histological examinations were performed by the same pathologist (R.M) who was blinded to all clinical and biological data. The following histopathological items were evaluated and scored semiquantitatively: 1) number of complete portal tracts; 2) number of central veins; 3) overall parenchymal architecture; 4) lobular inflammation; 5) central vein perivenulitis; 6) portal tract inflammation; 7) bile duct lesions; 8) bile duct loss; 9) presence of portal vein branches; 9) portal fibrosis; 10) perisinusoidal fibrosis. The detailed grading score employed is shown in Supplemental Table 1. For the semiquantitative assessment of iron content both Scheuer's modified and Total Iron Score (TIS) scoring systems were employed as described (Deugnier et al. Hepatology 1993; Scheuer et al. J Pathol Bacteriol 1962).

### Example 3. RNA extraction and processing.

For total RNA extraction cryopreserved liver tissue samples were homogenized in TRIzol reagent (Invitrogen, San Diego, CA, USA) using pestle and nuclease-free 1,5ml reaction tubes (Ambion). Total RNA was then extracted following the manufacturers guidelines and quality was assessed with the Agilent 2100 Bioanalyzer (Agilent Technologies, Santa Clara, USA).

### Example 4. Illumina microarray experiments.

One hundred and five liver RNA samples (20 TOL, 32 Non-TOL, 14 Non-TOL-Rej, 12 HEPC, 9 REJ, 8 CONT-Tx and 10 CONT; all of them from Hospital Clinic Barcelona) were processed into cRNA and hybridized onto Illumina HumanHT-12 Expression BeadChips containing 48,771 probes corresponding to 25,000 annotated genes (Illumina, Inc. San Diego, CA, USA). Expression data was computed using BeadStudio data analysis software (Illumina, Inc.) and subsequently processed employing quantiles normalisation using the Lumi bioconductor package [6]. Next, we conducted a conservative probe-filltering step excluding those probes with a coefficient of variation of 5%, which resulted in the selection of a total of 33,062 probes out of the original set of 48,771.

### Example 5. Affymetrix microarray experiments.

In a selected group of 10 TOL and 10 Non-TOL recipients microarray experiments were replicated onto Affymetrix Human Genome U133 Plus 2.0 arrays covering 47,000 annotated genes by 54,675 probes (Affymetrix, Inc, Santa Clara, CA, USA) and which comprises commercially available nucleic acid probes. Additional Affymetrix experiments were conducted in employing RNA extracted from 4 TOL-Post liver samples (4 of the 10 TOL recipients from whom liver biopsy tissue obtained 12 months after complete drug withdrawal was also available). Gene expression data were normalized using the guanidine-cytosine content-adjusted robust multiarray algorithm, which computes expression values from probe-intensities incorporating probe-sequence information. Thereupon, we employed a conservative probe-filtering step excluding probes not reaching a log₂ expression value of 5 in at least one sample, which resulted in the selection of a total of 18,768 probes out of the original set of 54,675.

### Example 6. Microarray gene expression data analysis.

To identify genes differentially expressed between the different microarray study groups we employed *Significant Analysis of Microarray* (SAM). SAM uses modified t test statistics for each gene of a dataset and a fudge factor to compute the t value, thereby controlling for unrealistically low standard deviations for each gene. Furthermore SAM allows control of the false discovery rate (FDR) by selecting a threshold for the difference between the actual test result and the result obtained from repeated permutations of the tested groups. For the current study we employed SAM selection using FDR<10% and 1000 permutations. To graphically represent global gene expression differences between the different study groups, the entire filtered probe list was used to perform a correspondence analysis as implemented in the *between-group-analysis* (BGA) function included in the *made4* package (Culhane AC, et al. Bioinformatics 2005). This method is capable of visualizing high-dimensional data such as multiple gene expression measurements in a 2D graph in which the areas delimited by the ellipses represent 95% of the estimated binormal distribution of the sample scores in the first and second axes. To determine whether the group of genes of interest was significantly associated with clinical outcome (tolerance versus rejection), clinical variables (donor and recipient age, gender, type of immunosuppressive therapy, time since transplantation) and histologic features (iron content), we employed the Globaltest software (Goeman, et al. Bioinformatics 2004). A syntax within this software was also employed to correct the association found between gene expression and clinical outcome for the possible confounding effects of nuisance clinical covariates found to be statistically different between tolerant and non-tolerant recipe
nts.

### Example 7. Quantitative real-time PCR (qPCR) experiments.

To validate the microarray expression results the expression patterns of a group of 104 target genes and 3 housekeeping genes (Supplementary Table 1) were measured employing the ABI 7900 Sequence Detection System and TaqMan LDA micro fluidic plates (Applied Biosystems, Carlsbad, USA), which comprises commercially available oligonucleotide primers, on a subgroup of 48 recipients (18 TOL and 31 Non-TOL; all of them from Hospital Clinic Barcelona). In addition, qPCR experiments were performed in an independent group of 10 TOL and 11 Non-TOL recipients provided by University Tor Vergata Rome and University Hospitals Leuven and from whom microarray data were not available. Target genes were selected based on: 1) Illumina and Affymetrix microarray experiment results; 2) blood transcriptional biomarkers previously described by our group as being associated with liver operational tolerance (M.Martinez-Llordella et al. J Clin Invest 2008); and 3) prominent immunoregulatory genes described in the literature. DNA was removed from total RNA preparations using Turbo DNA-free DNAse treatment (Ambion), and RNA was then reverse transcribed into cDNA using the HighCapacity cDNA Reverse Transcription Kit (Applied Biosystems). To quantify transcript levels target gene Ct values were normalized to the housekeeping genes to generate ΔCt values. The results were then computed as relative expression between cDNA of the target samples and a calibrated sample according to the ΔΔCt method. The following three samples were employed as calibrators: 1) pooled RNA from the 8 CONT-Tx samples; 2) pooled RNA from the 10 CONT samples; and 3) commercially available liver RNA *(Human Liver Total RNA,* Ambion).

### Example 8. Identification and validation of gene classifiers.

To develop biopsy-based qPCR gene expression classifiers to predict the success of immunosuppression withdrawal we conducted an exhaustive search for predictive models employing the linear discriminant analysis and logistic regression algorithms implemented in the *misclassification penalized posterior* (MiPP) software. MiPP is based on a stepwise incremental classification modelling for discovery of the most parsimonious diagnosis models and employs a double cross-validation strategy. First, to obtain the optimal models while avoiding the pitfalls of a large screening search, we conducted a 10-fold cross validation step on the training set of 18 TOL and 31 Non-TOL liver recipients from Hospital Clinic Barcelona. Next, random splitting cross-validation of the diagnosis models was conducted on the whole data set (which included the 56 samples from Barcelona and the 21 samples from Rome and Leuven) by repeatedly partitioning it into training set (2/3) and independent test set (1/3) for external model validation. For each model identified in the training set the optimal probability cut-off of tolerance was computed through a ROC analysis. The use of a large number of random splits of test and training sets allowed us to obtain confidence bounds on the accuracy of the diagnosis. On the basis of these confidence bounds, the diagnosis performance and mean misclassification error rates were obtained for each of the candidate classifiers. To demonstrate that the performance of the models was not center-dependent, we then computed SN, SP, NPV, PPV and overall error rates for the samples collected from Barcelona and those obtained from Rome and Leuven.

### Example 9. Serum hepcidine measurements.

Serum samples were obtained from the 64 enrolled liver recipients at baseline using BD vacutainer SST II (BD Bioscience, Franklin Lakes, USA) and stored at -80°C. Quantitative serum hepcidin measurements were conducted by a combination of weak cation exchange chromatography and time-of-flight mass spectrometry (TOF MS). For quantification a hepcidin analogue (synthetic hepcidin-24; Peptide International Inc.) was imployed as internal standard. Peptide spectra were generated on a Microflex LT matrix-enhanced laser desorption/ionisation TOF MS platform (Bruker Daltonics). Serum hepcidin-25 concentrations were expressed as nmol/L. The lower limit of detection of this method was 0.5 nM; average coefficients of variation were 2.7% (intra-run) and 6.5% (inter-run). The median reference level of serum hepcidin-25 is 4.2 nM, range 0.5- 13.9 nM.

### Example 10. Comparative assay between the method for diagnosis of tolerance in liver transplantation comprised in the state of the art and the method of the invention.

The method of the state of the art comprises the measure in peripheral blood samples of the expression of a group of genes *(KLRF1, PTGDR, NCALD, CD160, IL2RB, PTCH1, ERBB2, KLRB1, NKG7, KLRD1, FEZ1, GNPTAB, SLAMF7, CLIC3, CX3CR1, WDR67, MAN1A1, CD9, FLJ14213, FEM1C, CD244, PSMD14, CTBP2, ZNF295, ZNF267, RGS3, PDE4B, ALG8, GEMIN7)* different from that presented in the present invention. Moreover the present example shows that the method of the invention has a higher discriminative power.

The method of the state of the art gives rise to the following results:
A) Combination of *FEM1C* and *IL8:*
   SN=63%; SP=80.77%; ER= 7.08%; PPV=77.68%; NPV=72.41%
B) Combination of *KLRF1* and *SLAMF7*
   SN= 27.7%, SP 92.31%, ER= 37.5%, PPV=73.68%, NPV=72.41%
C) Combination of *KLRF1* and *IL2RB*
SN= 50%, SP= 84.62%, ER=31.25%, PPV= 73.33%, NPV=66.67%
SN: sensitivity
SP: specificity
ER: error rate
PPV: positive predictive value
NPV: negative predictive value

However, the method of the present invention based on the measure of the genes comprised in **Tables 3, 4 and 6** in liver tissue of the same 48 patients (the three best performing models were selected for this comparative assay) led to the following results:
A) Combination of *TFRC, CDHR2, HMOX1, MIF, HAMP, IFNG, PEBP1, SLC5A12, ADORA3:*
   SN=77.27%, SP=96.15%, ER=12.5%, PPV=94.44%, NPV=83.33%
B) Combination of *TFRC, PEBP1, MIF, ADORA3*
   SN=90.91%, SP=84,62%, ER=12.5%, PPV=83.33%, NPV=91.67%
C) Combination of *TFRC, IFNG, HAMP, CDHR2*
   SN=77.27%, SP=88.46%, ER=16.67%, PPV=85%, NPV=82.14%

SN: sensitivity
SP: specificity
ER: error rate
PPV: positive predictive value
NPV: negative predictive value

Therefore, it was concluded that the measurement of the expression of the genes comprised in the present invention in liver tissue samples, appears at least as accurate, than the measurement of the genes comprised in the state of the art in peripheral blood, in order to identify the liver recipients who can successfully leave the immunosuppressive medication because they are tolerant to the transplantation

### REFERENCES

1. Lerut, J., and Sanchez-Fueyo, A. 2006. An appraisal of tolerance in liver transplantation. Am J Transplant 6:1774-1780.
2. N. Najafian et al., "How can we measure immunologic tolerance in humans?" J. Am. Soc. Nephrol. 2006, vol. 17, pp. 2652-63.
3. Newell et al., "Tolerante assays: measuring the unknown", Transplantation 2006, vol. 81, pp. 1503-9.
4. J. Cai et al., "Minor H antigen HA-1-specific regulator and effector CD8+ T cells, and HA-1 microchimerism, in allograft tolerance", J. Exp. Med. 2004, vol. 199, pp. 1017-23.
5. E. Jankowska-Gan E et al., "Human liver allograft acceptance and the "tolerance assay", Hum. Immunol. 2002, vol. 63, pp. 862-70.
6. P. Sagoo et al., "Development of a cross-platform biomarker signature to detect renal transplant tolerance in humans", J.Clin. Invest. 2010, vol. 120, pp. 1848-61.
7. S. Brouard et al., "Operationally tolerant and minimally immunosuppressed kidney recipients display strongly altered blood T-cell clonal regulation", Am. J. Transplant. 2005, vol. 5, pp. 330-40.
8. G.V. Mazariegos et al., "Dendritic cell subset ratio in peripheral blood correlates with successful withdrawal of immunosuppression in liver transplant patients", Am. J. Transplant. 2003, vol. 3, pp. 689-96.
9. Y. Li et al., "Analyses of peripheral blood mononuclear cells in operational tolerance after pediatric living donor liver transplantation", Am. J. Transplant. 2004, vol. 4, pp. 2118-25.
10. M. Martinez-Llordella et al. Multiparameter of immune profiling of operational tolerance in liver transplantation. Am. J. Transplant. 2007, vol. 7, pp.309-19.
11. I.Puig-Pey et al. Characterization of gammadelta T cell subsets in organ transplantation. Transplant. Int. 2010. Epub.
12. S. Brouard et al. Identification of a peripheral blood transcriptional biomarker panel associated with operational renal allograft tolerance. Proc. Natl. Acad. Sci. U S A. 2007, vol. 104, pp. 15448-53.
13. K.A. Newell et al. Identification of a B cell signature associated with renal transplant tolerance in humans. J. Clin. Invest. 2010, vol. 120, pp. 1836-47.
14. Y.M. Deugnier et al. Differentiation between heterozygotes and homozygotes in genetic hemochromatosis by means of a histological hepatic iron index: a study of 192 cases. Hepatology 1993, vol. 17, pp. 30-4.
15. P.J. Scheuer et al. Hepatic pathology in relatives of patients with haemochromatosis. J. Pathol. Bacteriol. 1962, vol. 84, pp. 53-64.
16. A.C. Culhane et al. MADE4: an R package for multivariate analysis of gene expression data. Bioinformatics. 2005, vol. 21, pp. 2789-90.
17. J.J.Goeman et al. A global test for groups of genes: testing association with a clinical outcome. Bioinformatics. 2004, vol. 20, pp. 93-9.

## Claims

1. Method for the *in vitro* diagnosis or prognosis of the tolerant state of a patient subjected to a liver transplantation that comprises:
a. Measuring, in a biological sample obtained from the liver allograft of the patient under investigation, the expression level of at least one of the following genes or combinations thereof: *TFRC, CDHR2, HMOX1, MIF, HAMP, IFNG, PEBP1*, *SLC5A12*, *ADORA3* and *DAB2*;
b. Assessing the tolerance or non-tolerance of the patient under investigation to a liver transplantation by comparing the expression level of at least one of the genes or combinations thereof, of the step a), with the expression level of the same genes or combinations thereof, taken from a reference sample.

2. Method, according to claim 1, wherein the reference sample is a RNA sample is selected among: a pool of RNAs obtained from healthy non-transplanted liver tissue; a commercially available reference RNA; or an absolute reference RNA consisting in a sample containing a previously quantified number of RNA molecules.

3. Method, according to any of the claims 1 or 2, **characterized in that** the genes *TFRC* and *MIF* are up-regulated, and the genes *CDHR2*, *HMOX1*, *HAMP*, *IFNG, PEBP1, SLC5A12*, *ADORA3* and *DAB2* are down-regulated, in tolerant liver transplant recipients as compared with the expression level of the same genes taken from the reference RNA sample.

4. Method, according to any of the claims 1 to 3, **characterized in that** it comprises measuring the expression levels of at least one of the following gene combinations: *LC5A12*, *VNN3*, *TFRC*, *SOCS1*, *MIF*, *TTC3*, *RBM23*, *PEBP1*, *SH2DIB, NCR1, DAB2* and *ADORA3; TFRC, PEBP1, MIF, CDHR2, HAMP, TUBA4A, TTC3, HMOX1, VNN3, NCR1, ADORA3, TAF15, IFNG, SOCS1* and *TIPARP; MOX1, CDHR2, MIF, PEBP1, TFRC, SLC5A12, SOCS1, HAMP, VNN3* and *IFNG; TFRC, PEBP1, MIF, CDHR2, SLC5A12, HAMP, SOCS1, IFNG* and *HMOX1; TFRC, IFNG, CDHR2, ADORA3, HAMP, MIF, PEBP1, VNN3, SOCS1, HMOX1* and *DAB2; TFRC, DAB2, MIF, PEBP1, IFNG, HAMP, SLC5A12, SOCS1, VNN3, ADORA3, CDHR2, MCOLN1* and *HMOX1; TFRC, , IFNG, HMOX1, MCOLN1, MIF, HAMP, ADORA3, CDHR2, PEBP1* and *SOCSI; EBP1, TFRC, HMOX1, IFNG, MCOLN1, SOCSI, MIF, CDHR2, HAMP* and *ADORA3; TFRC, PEBP1, IFNG, CDHR2, ADORA3, VNN3, HMOX1, DAB2, SOCS1, MIF* and *HAMP; DHR2, ADORA3, IFNG, TFRC, VNN3, HMOX1, PEBP1, MIF, SLC5A12, HAMP, SOCS1* and *MCOLN1; LC5A12, TFRC, IFNG, MIF, DAB2, HMOX1, CDHR2, SOCS1, HAMP, PEBP1, VNN3, ADORA3* and *MCOLN1; TFRC, SOCS1, HMOX1, PEBP1, VNN3, CDHR2, HAMP, IFNG, DAB2, MCOLN1, ADORA3* and *MIF; TFRC, PEBP1, VNN3, SOCS1, MIF, HMOX1, DAB2, HAMP, IFNG, CDHR2, ADORA3* and *MCOLN1; LC5A12, MIF, CDHR2, TFRC, IFNG, ADORA3, HAMP, VNN3, SOCS1, MCOLN1, PEBP1* and *HMOX1; TFRC, IFNG, CDHR2, ADORA3, PEBP1, VNN3, MIF, HMOX1, MCOLN1, SOCS1, SLC5A12, DAB2* and *HAMP; TFRC, VNN3, HAMP, CDHR2, SLC5A12, HMOX1, SOCS1, PEBP1* and *MIF; AB2, TFRC, MIF, CDHR2, PEBP1, VNN3, TTC3, HMOX1* and *SOCS1; TFRC, PEBP1, MIF, CDHR2, VNN3, IFNG, MCOLN1* and *SOCS1; TFRC, PEBP1, MIF, SOCS1* and *CDHR2.*

5. Method, according to claims 1 to 3, wherein the expression level of the gene combination consisting of *TFRC, IFNG* and *CDHR2,* is measured.

6. Method, according to any of claim 1 to 5, which further comprises the determination of at least one additional parameter useful for the diagnosis or prognosis.

7. Method, according to claim 6, wherein this additional parameter is the serum level of Hepcidin.

8. Method, according to claim 6, wherein this additional parameter is the age and/or the time post-transplantation.

## Patentansprüche

1. Verfahren für die In-vitro-Diagnose- oder -Prognose des verträglichen Zustands eines Patienten, der einer Lebertransplantation unterzogen wird, welches umfasst:
a. Messung in einer biologischen Probe, die man von dem Leber-Allotransplantat des unter Überprüfung stehenden Patienten erhalten hat, des Expressionsniveaus von mindestens einem der folgenden Gene oder Kombinationen oder Äquivalente davon: TFRC, CDHR2, HMOX1, MIF, HAMP, IFNG, PEBP1, SLC5A12, ADORA3 und DAB2;
b. Beurteilung der Verträglichkeit oder Unverträglichkeit des unter Überprüfung stehenden Patienten gegenüber einer Lebertransplantation durch den Vergleich des Expressionsniveaus von mindestens einem der Gene oder Kombinationen davon, aus Schritt a), mit dem Expressionsniveau der gleichen Gene oder Kombinationen davon, die von einer Referenzprobe genommen werden.

2. Verfahren nach Anspruch 1, wobei die Referenzprobe eine RNA-Probe ist, die ausgewählt wurde unter: einem Pool von RNAs, die man von gesundem, nicht-transplantiertem Lebergewebe erhalten hat; einer kommerziell erhältlichen Referenz-RNA; oder einer absoluten Referenz-RNA bestehend aus einer Probe, die eine vorher quantifizierte Anzahl von RNA-Molekülen enthält.

3. Verfahren nach einem der Ansprüche 1 oder 2, **dadurch gekennzeichnet, dass** die Gene TFRC und MIF nach oben reguliert werden und die Gene CDHR2, HMOX1, HAMP, IFNG, PEBP1, SLC5A12, ADORA3 und DAB2 nach unten reguliert werden, bei Lebertransplantatempfängern mit Verträglichkeit im Vergleich mit dem Expressionsniveau der gleichen Gene, die von der Referenz-RNA-Probe genommen werden.

4. Verfahren nach einem der Ansprüche 1 bis 3, **dadurch gekennzeichnet, dass** es die Messung der Expressionsniveaus von mindestens einer der folgenden Genkombinationen umfasst: LC5A12, VNN3, TFRC, SOCS1, MIF, TTC3, RBM23, PEBP1, SH2D1B, NCR1, DAB2 und ADORA3; TFRC, PEBP1, MIF, CDHR2, HAMP, TUBA4A, TTC3, HMOX1, VNN3, NCR1, ADORA3, TAF15, IFNG, SOCS1 und TIPARP; MOX1, CDHR2, MIF, PEBP1, TFRC, SLC5A12, SOCS1, HAMP, VNN3 und IFNG; TFRC, PEBP1, MIF, CDHR2, SLC5A12, HAMP, SOCS1, IFNG und HMOX1; TFRC, IFNG, CDHR2, ADORA3, HAMP, MIF, PEBP1, VNN3, SOCS1, HMOX1 und DAB2; TFRC, DAB2, MIF, PEBP1, IFNG, HAMP, SLC5A12, SOCS1, VNN3, ADORA3, CDHR2, MCOLN1 und HMOX1; TFRC, IFNG, HMOX1, MCOLN1, MIF, HAMP, ADORA3, CDHR2, PEBP1 und SOCS1; EBP1, TFRC, HMOX1, IFNG, MCOLN1, SOCS1, MIF, CDHR2, HAMP und ADORA3; TFRC, PEBP1, IFNG, CDHR2, ADORA3, VNN3, HMOX1, DAB2, SOCS1, MIF und HAMP; DHR2, ADORA3, IFNG, TFRC, VNN3, HMOX1, PEBP1, MIF, SLC5A12, HAMP, SOCS1 und MCOLN1; LC5A12, TFRC, IFNG, MIF, DAB2, HMOX1, CDHR2, SOCS1, HAMP, PEBP1, VNN3, ADORA3 und MCOLN1; TFRC, SOCS1, HMOX1, PEBP1, VNN3, CDHR2, HAMP, IFNG, DAB2, MCOLN1, ADORA3 und MIF; TFRC, PEBP1, VNN3, SOCS1, MIF, HMOX1, DAB2, HAMP, IFNG, CDHR2, ADORA3 und MCOLN1; LC5A12, MIF, CDHR2, TFRC, IFNG, ADORA3, HAMP, VNN3, SOCS1, MCOLN1, PEBP1 und HMOX1; TFRC, IFNG, CDHR2, ADORA3, PEBP1, VNN3, MIF, HMOX1, MCOLN1, SOCS1, SLC5A12, DAB2 und HAMP; TFRC, VNN3, HAMP, CDHR2, SLC5A12, HMOX1, SOCS1, PEBP1 und MIF; AB2, TFRC, MIF, CDHR2, PEBP1, VNN3, TTC3, HMOX1 und SOCS1; TFRC, PEBP1, MIF, CDHR2, VNN3, IFNG, MCOLN1 und SOCS1; TFRC, PEBP1, MIF, SOCS1 und CDHR2.

5. Verfahren nach den Ansprüchen 1 bis 3, wobei das Expressionsniveau der Genkombination bestehend aus TFRC, IFNG und CDHR2 gemessen wird.

6. Verfahren nach einem der Ansprüche 1 bis 5, welches ferner die Bestimmung von mindestens einem zusätzlichen Parameter, der für die Diagnose oder Prognose nützlich ist, umfasst.

7. Verfahren nach Anspruch 6, wobei dieses zusätzliche Parameter der Serumspiegel von Hepcidin ist.

8. Verfahren nach Anspruch 6, wobei dieses zusätzliche Parameter das Alter und/oder die Zeit nach der Transplantation ist.

## Revendications

1. Procédé pour le diagnostic ou pronostic *in vitro* de l'état de tolérance d'un patient soumis à une transplantation hépatique qui comprend :
a) La mesure, dans un échantillon biologique obtenu de l'allogreffe de foie du patient sous observation, du niveau d'expression d'au moins un des gènes suivants ou de leurs combinaisons : TFRC, CDHR2, HMOX1, MIF, HAMP, IFNG, PEBP1, SLC5A12, ADORA3 et DAB2 ;
b) l'évaluation de la tolérance ou non-tolérance du patient sous observation d'une transplantation hépatique en comparant le niveau d'expression d'au moins un des gènes ou de leurs combinaisons, de l'étape a), au niveau d'expression des mêmes gènes ou de leurs combinaisons, prélevés dans un échantillon de référence.

2. Procédé, selon la revendication 1, dans lequel l'échantillon de référence qui est un échantillon d'ARN est sélectionné parmi : un pool d'ARNs obtenus dans un tissu hépatique sain non transplanté ; un ARN de référence disponible dans le commerce ou un ARN de référence absolue consistant en un échantillon contenant un nombre préalablement quantifié de molécules d'ARN.

3. Procédé, selon n'importe laquelle des revendications 1 ou 2, **caractérisé en ce que** les gènes TFRC et MIF sont régulés à la hausse, et les gènes CDHR2, HMOX1, HAMP, IFNG, PEBP1, SLC5A12, ADORA3 et DAB2 sont régulés à la baisse, chez des receveurs tolérants de transplant hépatique par comparaison avec le niveau d'expression des mêmes gènes prélevés dans l'échantillon de référence d'ARN.

4. Procédé, selon n'importe laquelle des revendications 1 à 3, **caractérisé en ce qu'**il comprend la mesure des niveaux d'expression d'au moins une des combinaisons de gènes suivantes: LC5A12, VNN3, TFRC, SOCS1, MIF, TTC3, RBM23, PEBP1, SH2D1B, NCR1, DAB2 et ADORA3 ; TFRC, PEBP1, MIF, CDHR2, HAMP, TUBA4A, TTC3, HMOX1, VNN3, NCR1, ADORA3, TAF15, IFNG, SOCS1 et TIPARP ; MOX1, CDHR2, MIF, PEBP1, TFRC, SLC5A12, SOCS1, HAMP, VNN3 et IFNG ; TFRC,PEBP1, MIF, CDHR2, SLC5A12, HAMP, SOCS1, IFNG, et HMOX1 ; TFRC, IFNG, CDHR2, ADORA3, HAMP, MIF, PEBP1, VNN3, SOCS1, HMOX1 et DAB2 ; TFRC, DAB2, MIF, PEBP1, IFNG, HAMP, SLC5A12, SOCS1, VNN3, ADORA3, CDHR2, MCOLN1 et HMOX1 ; TFRC, IFNG, HMOX1, MCOLN1, MIF, HAMP, ADORA3, CDHR2, PEBP1 et SOCS1 ; EBP1, TFRC, HMOX1, IFNG, MCOLN1, SOCS1, MIF, CDHR2, HAMP et ADORA3 ; TFRC, PEBP1, IFNG, CDHR2, ADORA3, VNN3, HMOX1, DAB2, SOCS1, MIF et HAMP ; DHR2, ADORA3, IFNG, TFRC, VNN3, HMOX1, PEBP1, MIF, SLC5A12, HAMP, SOCS1 et MCOLN1 ; LC5A12, TFRC, IFNG, MIF, DAB2, HMOX1, CDHR2, SOCS1, HAMP, PEBP1, VNN3, ADORA3 et MCOLN1 ; TFRC, SOCS1, HMOX1, PEBP1, VNN3, CDHR2, HAMP, IFNG, DAB2, MCOLN1, ADORA3 et MIF ; TFRC, PEBP1, VNN3, SOCS1, MIF, HMOX1, DAB2, HAMP, IFNG, CDHR2, ADORA3 et MCOLN1 ; LC5A12, MIF, CDHR2, TFRC, IFNG, ADORA3, HAMP, VNN3, SOCS1, MCOLN1, PEBP1 et HMOX1 ; TFRC, IFNG, CDHR2, ADORA3, PEBP1, VNN3, MIF, HMOX1, MCOLN1, SOCS1, SLC5A12, DAB2 et HAMP ; TFRC, VNN3, HAMP, CDHR2, SLC5A12, HMOX1, SOCS1, PEBP1 et MIF ; AB2, TFRC, MIF, CDHR2, PEBP1, VNN3, TTC3, HMOX1 et SOCS1 ; TFRC, PEBP1, MIF, CDHR2, VNN3, IFNG, MCOLN1 et SOCS1 ; TFRC, PEBP1, MIF, SOCS1 et CDHR2.

5. Procédé, selon les revendications 1 à 3, dans lequel le niveau d'expression de la combinaison de gènes constituée de TFRC, IFNG et CDHR2, est mesuré.

6. Procédé, selon n'importe laquelle des revendications 1 à 5, qui comprend en outre la détermination d'au moins un paramètre supplémentaire utile pour le diagnostic ou pronostic.

7. Procédé, selon la revendication 6, dans lequel ce paramètre supplémentaire est le taux sérique d'Hepcidine.

8. Procédé, selon la revendication 6, dans lequel ce paramètre supplémentaire est l'âge et/ou le temps post-transplantation.
